# EUROPEAN PATENT SPECIFICATION

(11) **EP 3 747 380 B1**
(45) Date of publication and mention of the grant of the patent: **22.06.2022**
(21) Application number: 20178387.5
(22) Date of filing: 04.06.2020
(51) Int. Cl.: A61B 17/3207, A61B 17/00

(54) **APPARATUS FOR BREAKING THROMBUS AND ASPIRATING THROMBUS**
VORRICHTUNG ZUM BRECHEN UND ANSAUGEN VON THROMBUS
APPAREIL DE RUPTURE ET D'ASPIRATION DU THROMBUS

(30) Priority: 06.06.2019 CN 201910490706
(43) Date of publication of application: 09.12.2020
(73) Proprietor: Sinomed Neurovita Technology Inc., Suzhou, Jiangsu Province (CN)
(72) Inventor: MIAO, ZHONGRONG, TEDA, Tianjin 300457 (CN); LI, ZHONGHUA, TEDA, TIANJIN 300457 (CN); LI, TIANZHU, TEDA, TIANJIN 300457 (CN); MA, JIANXIANG, TEDA, TIANJIN 300457 (CN); LI, JING, TEDA, TIANJIN 300457 (CN)
(74) Representative: Regimbeau

(56) References cited:
- EP-A1- 0 315 290
- WO-A1-00/51504
- US-A1- 2003 216 760
- US-A1- 2017 020 556
- US-A1- 2018 064 462
- US-A1- 2018 228 502

## Description

### Technical field

The invention relates to the field of medical devices, particularly to an apparatus for breaking thrombus and aspirating the thrombus.

### Background

Stroke is one of the main causes of human disability and death. Acute ischemic stroke (AIS) accounts for about 80% of all stroke. The key point of AIS treatment is to dredge the occluded blood vessel and restore the blood flow as early as possible to save the ischemic penumbra. At present, the typical treatment includes intravenous thrombolysis and mechanical thrombectomy.

Intravenous thrombolysis method is to inject thrombolytic agent into the vein, the main component of which is tissue plasminogen activator (rt-PA). The principle is that rt-PA can dissolve the thrombus in the diseased blood vessel, so as to achieve the purpose of recanalization of blood flow in the diseased blood vessel, such that the cerebral tissue in the vascular perfusion area can get the blood oxygen supply again, and thus reduce the area of cerebral infarction. However, the treatment of intravenous thrombolysis has strict time limit, generally within 4.5 hours of stroke onset; and some drugs are usually delivered through systemic drug release, which will increase the risk of systemic hemorrhage including cerebral hemorrhage, such that the intravenous thrombolysis is only applicable to the treatment of limited individuals.

Another option is to use instruments for thrombectomy. There are two kinds of interventional thrombectomy instruments available on the market at present. One is a suction catheter, which is designed as a structure similar to that of a micro catheter: after arriving at the lesion, the distal end of the suction catheter is applied with a negative pressure, and the free thrombus blocking the blood vessel is inhaled into the catheter or is suctioned to the catheter port and then is pulled out of the body, so as to restore the blood flow at the lesion. The other is thrombectomy stent, and the principle of stent thrombectomy is to use a micro catheter technology, the stent thrombectomy device reaches the intracranial artery along the lower limb artery through the internal artery channel, then thrombectomy means at the anterior segment of the catheter actively "catches" the thrombus which blocks the blood vessel, and a negative pressure is applied at the proximal end of the micro catheter, so that the thrombus is extracted out of the human body. The main advantage of mechanical thrombectomy is that it expands the time window of treatment to 4.5-10 hours after the onset of the disease. However, at present, there are some shortcomings in mechanical thrombectomy, such as when the device takes thrombus out of the human body, some blood clots or particles may fall during the process of retrieval or the device cannot completely remove thrombus due to the complexity of thrombus.

Therefore, it is necessary to develop a thrombus removal device which can not only expand the time window, but also take thrombus out of human body completely, so as to solve the existing problems of thrombus removal with instruments.

The information disclosed in the background part of the invention is only intended to strengthen the understanding of the general background technology of the invention, and shall not be deemed to recognize or imply in any form that the information constitutes the prior art known to those skilled in the art.

US 2003/216760 A1 discloses a device for removal of obstructions formed in a lumen according to the preamble.

US 2017/020556 A1 discloses an apparatus and method for removing a clot from a patient's vascular system.

WO 00/51504 A1 discloses a rotational atherectomy system with a serrated cutting tip.

EP 0 315 290 A1 discloses an atherectomy catheter useful in restoring patency to a blood vessel that is blocked or partially blocked by atheromas or other form of stenotic or thrombotic lesion.

US 2018/064462 A1 discloses endovascular devices.

US 2018/228502 A1 discloses the design and use of clot aspiration catheters for use in the coronary, peripheral, and neuro vasculature.

### Summary

The invention is defined in claim 1. Further embodiments are defined in the dependent claims.

The object is to provide an apparatus for breaking thrombus and aspirating the thrombus. The aspirating action is carried out synchronously with mechanical thrombus breaking, with a high dredging rate (> 98%) and significant reduction of complications.

Another object is to provide an apparatus for breaking thrombus and aspirating the thrombus. The mechanical rotary cutting is integrated in the micro catheter, which can effectively reduce the iatrogenic damage to the blood vessel wall in the process of extracting thrombus.

Another object is to provide an apparatus for breaking thrombus and aspirating the thrombus, whose distal end and sides are all provided with thrombus breaking and aspirating ports, so as to significantly improve the efficiency of extracting thrombus.

The beneficial effects are as follows:
1. The aspirating action is carried out synchronously with the mechanical thrombus breaking, with a high dredging rate (> 98%) and significant reduction of complications.
2. Syringe is connected directly for aspiration without configuration of aspirating equipment, and the doctor can complete the thrombus aspiration and mechanical extracting actions (both are carried out synchronously) with one hand operation.
3. Integrated battery supplies power, which needs no external power supply and is convenient for operation.
4. Mechanical rotary cutting is integrated into a micro catheter, which can effectively reduce the iatrogenic damage to the blood vessel wall in the process of thrombectomy.
5. The distal end and the sides are all equipped with thrombus breaking ports and aspirating ports and thus significantly improve the efficiency of thrombus extraction.
6. Thrombus has been smashed at the same time of aspiration, so it will not block the aspirating catheter, and the efficiency of thrombus extraction is high.
7. Mechanical rotary cutting is helpful to capture thrombus which has been formed for a long time and thus expand the window period of thrombus extraction.
8. There is a blood flow seal at the proximal end to prevent the embolus from rushing to the distal end by blood flow.
9. This technology can be extended to the fields of coronary artery thrombectomy, peripheral thrombectomy and pulmonary embolism.

### Brief description of drawings

Fig. 1, Fig. 2 and Fig. 3 are the overall structural schematic diagrams of three kinds of apparatuses for breaking thrombus and aspirating the thrombus according to embodiments of the invention, respectively.
Fig. 4 to Fig. 10 are the structural schematic diagrams of a distal end rotary grinding head device according to embodiments of the invention.
Fig. 11 is an assembly diagram of a connection mandrel and a stabilizer according to embodiments of the present invention.
Figs. 12 to 15 are structural schematic diagrams of four kinds of connecting spindles according to embodiments of the invention.
Fig. 16 and Fig. 17 are schematic diagrams of a special shape of a connection mandrel according to embodiments of the present invention.
Fig. 18 to Fig. 22 are schematic diagrams of the shape of the rotary grinding head opening of the distal end rotary grinding head device according to embodiments of the present invention.
Fig. 23 is a structural schematic diagram of a micro catheter device according to embodiments of the present invention.
Figs. 24 to 27 are structural schematic diagrams of a distal end head of the micro catheter device according to embodiments of the present invention.
Fig. 28 to Fig. 30 are schematic diagrams of the assembly of a micro catheter device and a distal end rotary grinding head device according to embodiments of the invention.
Figs. 31 and 32 are schematic diagrams of a distal end structure of a micro catheter device according to embodiments of the present invention.

### Reference numerals:

1-distal end rotary grinding head device
2-micro catheter device
3-aspirating device
4-rotary grinding head driving device
5-connection mandrel
6-aspirating rotary grinding hand-held system
7-waste liquid collecting device
8-power supply device
9-control device
10-check device
11-blocking device
12-handle housing
13-reset button
14-drive mechanism
15-pull rod
16-reset spring
17-press rod
18-spring contact
19-plug
20-handle
21-copper sheet
22-side surface
23-rotary grinding head opening
24-serrated edge
25-wavy edge
26-tooth edge
27-irregular tooth edge
28- proximal end interface
29-spiral filiform structure
30-distal end
31-micro catheter opening
32-non occluded head
33, 34-semi occluded limit structure
35-fully occluded limit structure
36-opening structure
37-rotary cutting head
38-top point of the serrated edge of micro catheter opening
39- bottom point of the serrated edge of micro catheter opening
40-top point of the serrated edge of rotary grinding head opening
41- bottom point of the serrated edge of rotary grinding head opening
42-moving device
43-rotary grinding tip
44-stabilizer
45-protective sheath tube
46-core wire
47-cutting pattern.

It should be understood that the appended drawings are not drawn to scale, but merely to illustrate a properly simplified representation of the various features of the basic principles of the present invention. The specific design features of the present invention disclosed herein include, for example, specific dimensions, directions, positions and shapes to be determined in part by the specific application and use environment. In the attached drawings, the same or equivalent parts (elements) are marked with the same reference numerals.

### Detailed description of embodiments

The disclosure will be further described in combination with embodiments and corresponding figures.

The disclosure relates to an apparatus for breaking and aspirating thrombus and a manufacturing method thereof.

Any mechanical thrombus extraction apparatus which can be purchased on the market can be used to manufacture the apparatus of the disclosure. For example, such apparatuses include but are not limited to the following:
1) Penumbra System ^{®} is manufactured by Penumbra, Inc. A detailed description of its apparatus is provided in the patent (Patent No.: US20180353194A1, US20180263646A1).
2) ROTABLATOR^{™} and ROTAPRO^{™} Rotational Atherectomy Systems, are manufactured by Boston Scientific Corporation. The detailed specification of its apparatus is provided in the patent (Patent No.: US6077282A).

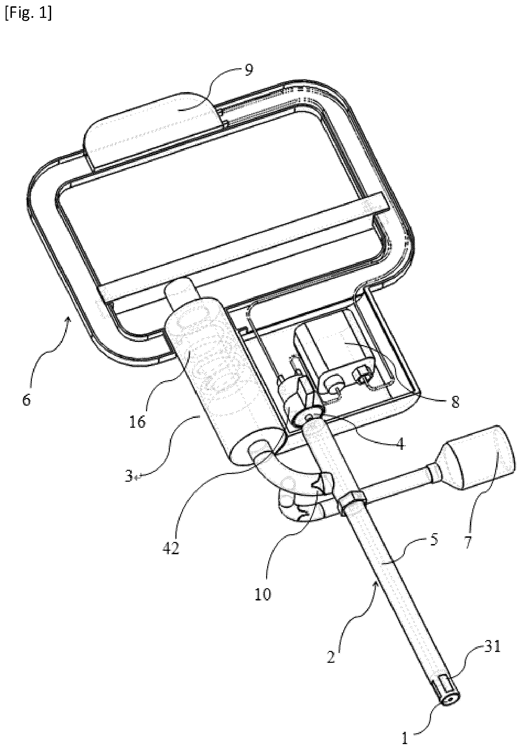

Fig. 1 is an overall structural schematic diagram of an apparatus for breaking thrombus and aspirating the thrombus according to an embodiment of the invention. The apparatus for breaking thrombus and aspirating the thrombus comprises an aspirating rotary grinding hand-held system 6, a micro catheter device 2, a waste liquid collecting device 7 and a distal end rotary grinding head device 1, wherein the distal end rotary grinding head device 1 cooperate with the micro catheter device 2, and the distal end rotary grinding head device 1 is connected with a rotary grinding head driving device 4 through a connection mandrel 5, and the rotary grinding head driving device 4 is driven by a power supply device 8, generating torque to drive the distal end rotary grinding head device 1 to rotate at high speed, and cooperate with the micro catheter device 2 to complete the breaking action of the obstacles (thrombus) in the blood vessel. In order to achieve a good thrombus cutting effect, the difference between the size of the dsital end rotary grinding head device 1 and the micro catheter device 2 (that is, the outer diameter of the distal end rotary grinding head device 1 and the inner diameter of the micro catheter device 2) should not exceed 0.05 mm. The operator can operate the moving device 42 to control the position of the distal end rotary grinding head device 1 in the micro catheter device 2, and the sliding distance of the distal end rotary grinding head device 1 is between 1 mm and 50 mm, so as to ensure that the relative position of the distal end rotary grinding head device 1 and the micro catheter opening 31 of the micro catheter device 2 will not be changed in the highly curved blood vessel, which will affect the effect of thrombus rotary cutting. The length of the distal end rotary grinding head device 1 beyond the micro catheter device 2 ranges between 0.5 mm and 5 mm, so that the distal end rotary grinding head device 1 can directly rotationally grind thrombus, which achieves better crushing effect without damaging blood vessels.

The aspirating device 3 generates enough negative air pressure through manual pressure gripping or electric energy drive, which is used to aspirate the broken thrombus or the blood clots or particles falling or broken in the process of breaking the thrombus into the external liquid waste collecting device 7, so as to prevent them from moving to farther region of the blood vessels in the body; at the same time, a check device 10 is arranged at the connection between the aspirating device 3 and the liquid waste collecting device 7, which can ensure that the broken thrombus always flows in the direction of the waste liquid collecting device 7 from the micro catheter device 2 to the aspirating device 3, without counter-flow. The aspirating device 3 and the rotary grinding head driving device 4 are integrated in the aspirating rotary grinding hand-held system 6, and the aspirating device 3 and the rotary grinding head driving device 4 are connected together through a control device 9, and the connection way can be circuit connection or mechanical connection, so as to realize the function that the rotary grinding process and the aspirating process conduct simultaneously, and facilitate the single hand operation of the operator.

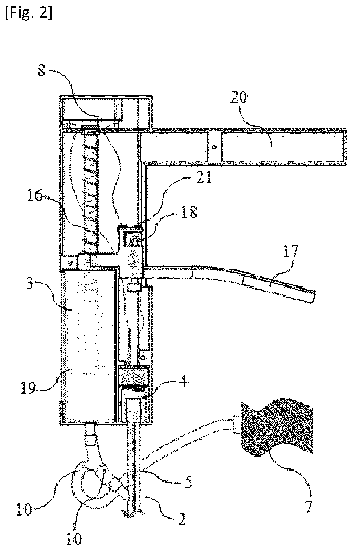

Fig. 2 is an overall structural schematic diagram of an apparatus for breaking thrombus and aspirating the thrombus according to an embodiment of the present invention. During use, the operator holds the press rod 17 and the handle 20; the palm presses the press rod 17, so that the press rod 17 gets close to the handle 20. The spring contact 18 contacts with the copper sheet 21 to form a circuit connection, so that the rotary grinding head driving device 4 starts to operate; at the same time, the plug 19 is driven close to the handle 20, so that the aspirating device 3 generates a negative pressure, and the thrombus in the blood vessel is aspirated into the aspirating device 3, and then collected into the waste liquid collecting device 7.

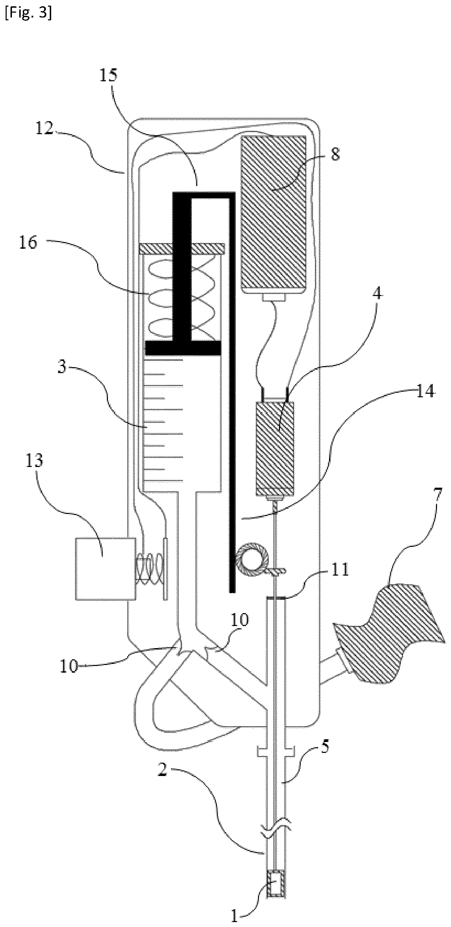

Fig. 3 is an overall structural schematic diagram of an apparatus for breaking thrombus and aspirating the thrombus according to an embodiment of the present invention. When the operator holds the handle housing 12 with one hand and presses the reset button 13, the power supply device 8 supplies power to the rotary grinding head driving device 4. The rotary grinding head driving device 4 drives the drive mechanism 14 to rotate, and the pull rod 15 in the aspirating device 3 moves away from the reset button 13 under the driving of the drive mechanism 14, such that vacuum establishes in the aspirating device 3, and the generated aspiration force can aspirate the thrombus from the blood vessel into the micro catheter device 2. The connection mandrel 5 is connected with the rotary grinding head driving device 4, and the connection way can be laser welding, glue bonding or other mechanical structure fastening. The connection mandrel 5 drives the distal end rotary grinding head device 1 to rotate at a high speed, breaking all thrombus aspirated into the micro catheter device 2. The broken thrombus is aspirated into the aspirating device 3. The blocking device 11 is located in the micro catheter close to the motor side. It ensures that the liquid path is always in a vacuum state and maximizes the aspirating force. When the operator releases the button, the reset spring 16 pushes the pull rod 15 back to the initial position, and thus pushes the thrombus and blood in the aspirating device 3 to the liquid waste collecting device 7. The check device (here, implemented as a one-way valve) 10 can ensure that the thrombus and blood always flow in one-way along the direction from the micro catheter device 2 to the aspirating device 3 to the liquid waste collecting device 7.

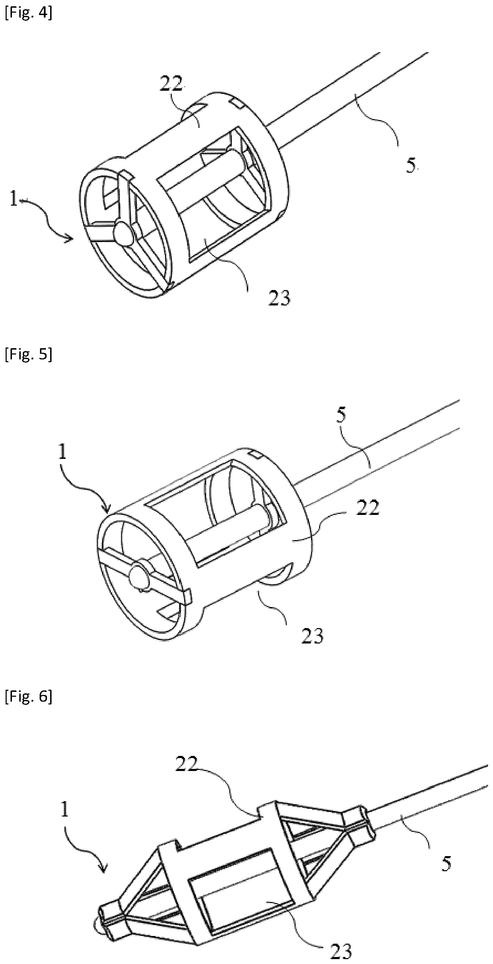

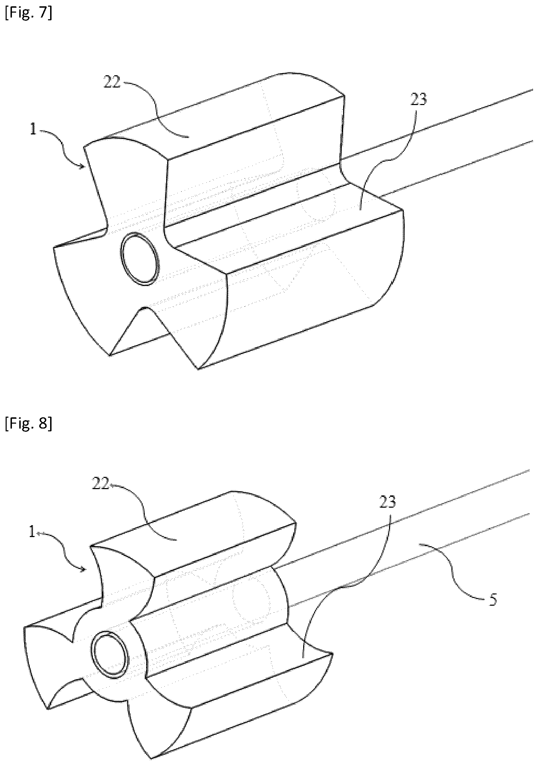

Figs. 4 to 10 show the distal end rotary grinding head device 1 and the connection mandrel 5. The shape of the distal end rotary grinding head device 1 can be cylindrical or shuttle shaped, and it can be an integrated structure or it can be formed by connecting multiple parts. The connection way of the multiple parts can be adhesive, laser welding or mechanical connection. The side surface 22 of the distal end rotary grinding head device 1 is provided with rotary grinding head openings 23. The connection mandrel 5 can be a metal wire, which can be made of metal materials with good biocompatibility, and the metal should have good kink resistance. The distal end of the distal end rotary grinding head device 1 can have a rotary grinding tip 43, which can be beyond the scope of the micro catheter device 2 to directly contact with the thrombus, so as to having better performance of rotary grinding. At the same time, the round head design of the rotary grinding tip 43 will not scratch the blood vessels, ensuring the safety.

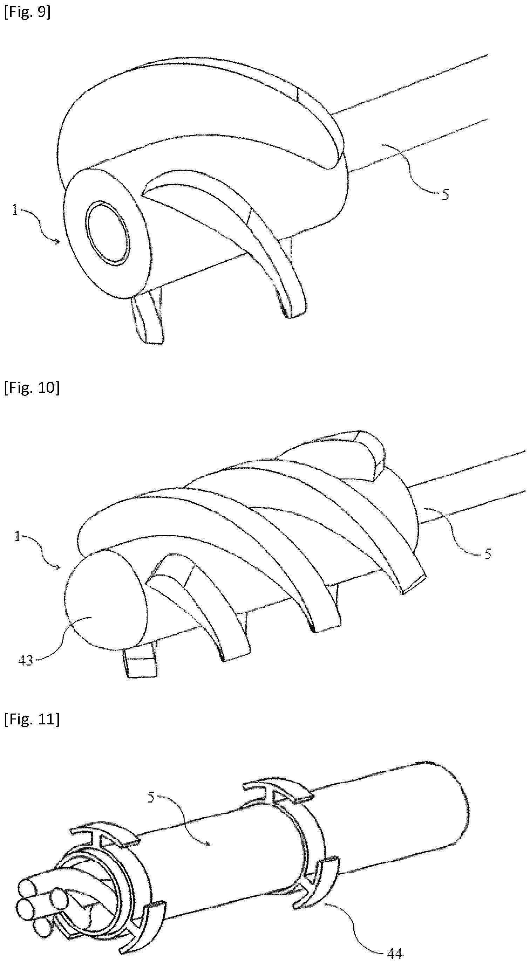

A stabilizer 44 can be installed on the outer surface of the connection mandrel 5, as shown in Fig. 11. The width of the stabilizer 44 is between 1 mm and 3 mm, and the number is between 2 and 15. The stabilizer 44 and the connection mandrel 5 can be bonded or welded together. The stabilizer 44 is clearance fitted with the inner surface of the micro catheter device 2, so as to ensure that the connection mandrel 5 does not rub against the inner surface of the micro catheter device 2 when rotating, and to prevent the connection mandrel 5 from twisting. The material of the stabilizer 44 can be hard alloy or polymer material.

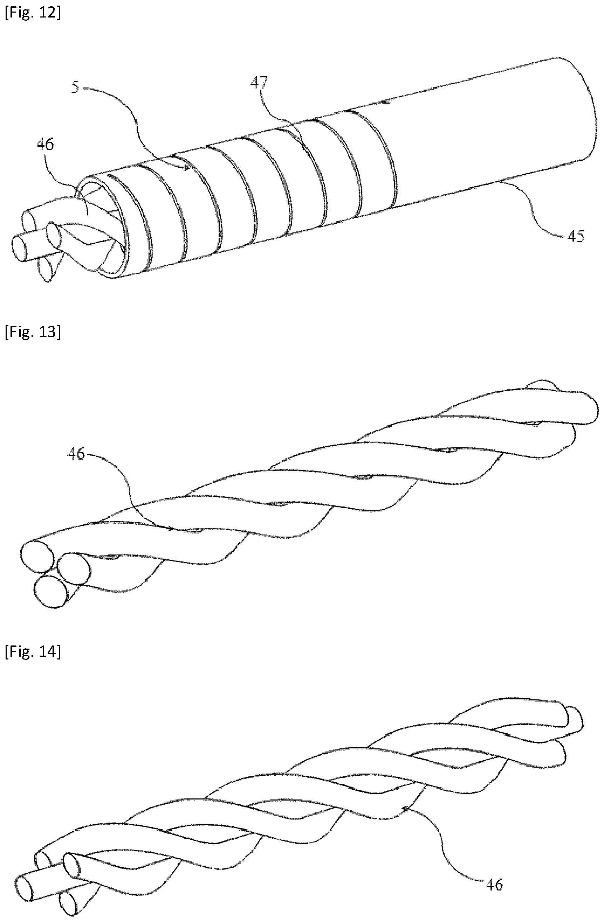

Fig. 12 shows a structure of the connection mandrel 5, wherein the core wire 46 passes through the protective sheath tube 45, so that the connection mandrel 5 has better rigidity and thus is more conducive to the transmission of torque to the distal end rotary grinding head device. The length of the protective sheath tube 45 is shorter than that of the core wire 46, and the difference between the two lengths is 2 mm to 150 mm. The material for protecting sheath tube 45 may be hard alloy, super elastic metal or polymer material. The surface of the protective sheath tube 45 can have a spiral cutting pattern 47, further increasing the flexibility of the protective sheath tube 45, so that the micro catheter device 2 can better fit the blood vessel.

Fig. 13 and Fig. 14 show the structure of core wire 46, which can be formed by winding and weaving multiple metal threads, or by winding and weaving multiple metal threads around one metal thread, and the number of metal thread is between 2 and 12.

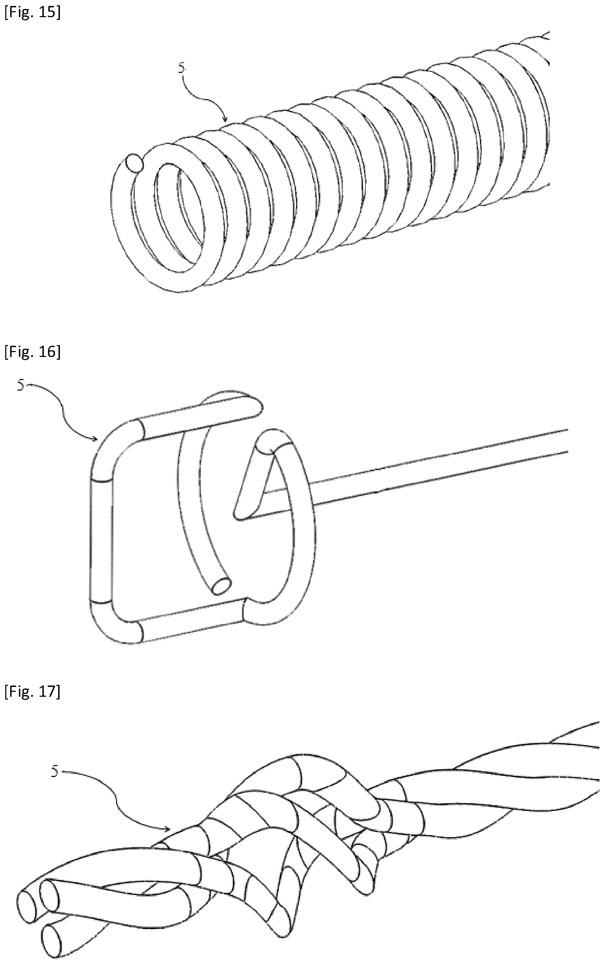

In addition, the connection mandrel 5 also can be, as shown in Fig. 15, Fig. 16 and Fig. 17, is spirally bent into a spring shape from a single strand of metal wire (as shown in Fig. 15), and the cross section of the metal wire can be circular or rectangular. Or one end of the connection mandrel 5 is bent or expanded to form a special shape (as shown in Fig. 16 and Fig. 17), which plays the role of rotary cutting, so as to replace the distal end rotary grinding head device 1. The special shape part of the connection mandrel 5 can be reinforced by adhesive or laser welding.

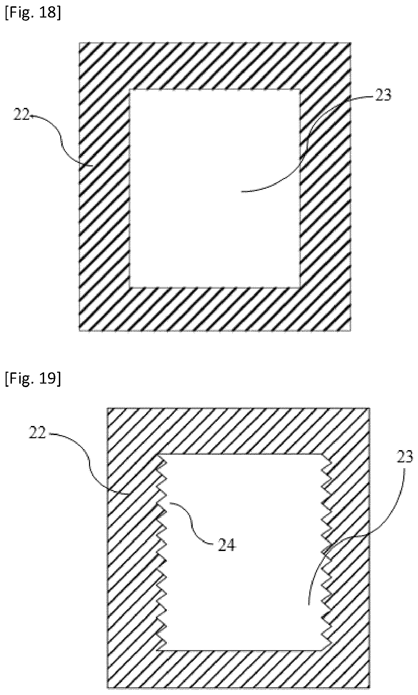

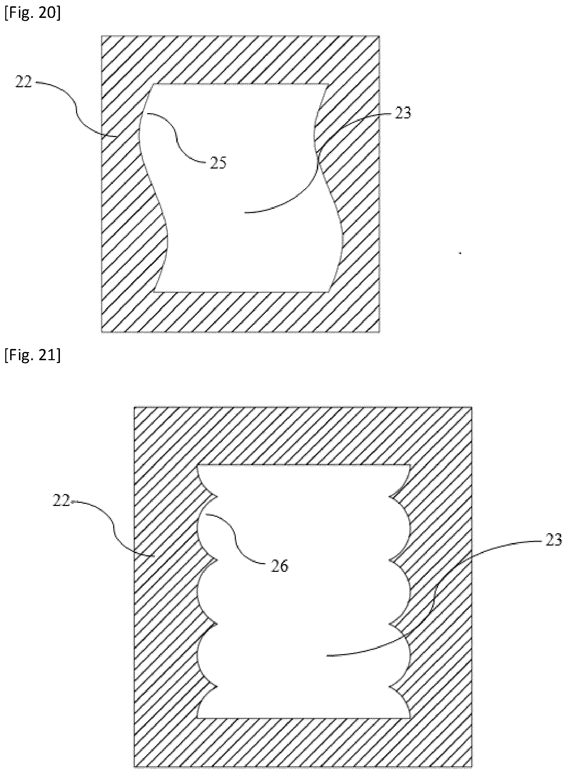

The rotary grinding head openings 23 shown in Figs. 18 to 22 can be rectangular in shape, or contain a serrated edge 24, a wavy edge 25, a tooth edge 26 or an irregular tooth edge 27 along the length direction of the catheter.

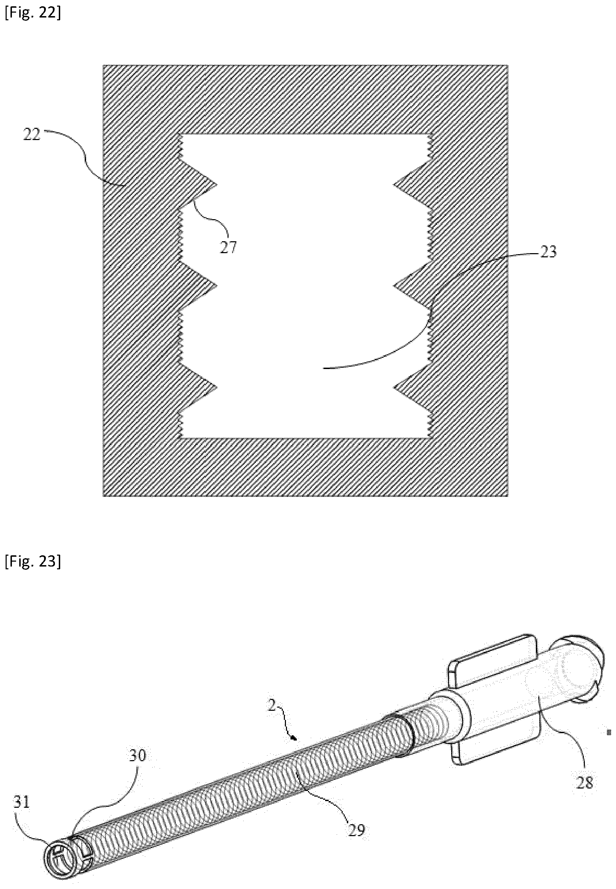

Fig. 23 shows the structure of the micro catheter device 2 according to an embodiment of the present invention, wherein the micro catheter device 2 can optionally include a proximal end interface 28 which can be quickly attached and detached; of course, the proximal end interface 28 can also be excluded, and the micro catheter device 2 and the aspirating rotary grinding hand-held system 6 are integral and cannot be disassembled.

The metal spiral filiform structure 29 is embedded in the micro catheter device 2, or the metal spiral filiform structure 29 is within the diameter range of the micro catheter device 2, and they cooperate with each other. The distal end head 30 of the micro catheter device 2 is provided with micro catheter openings 31. The micro catheter device 2 is made of both polymer and metal materials with good biocompatibility, whose inner wall needs to be smooth and does not deform when passing through tortuous vessels.

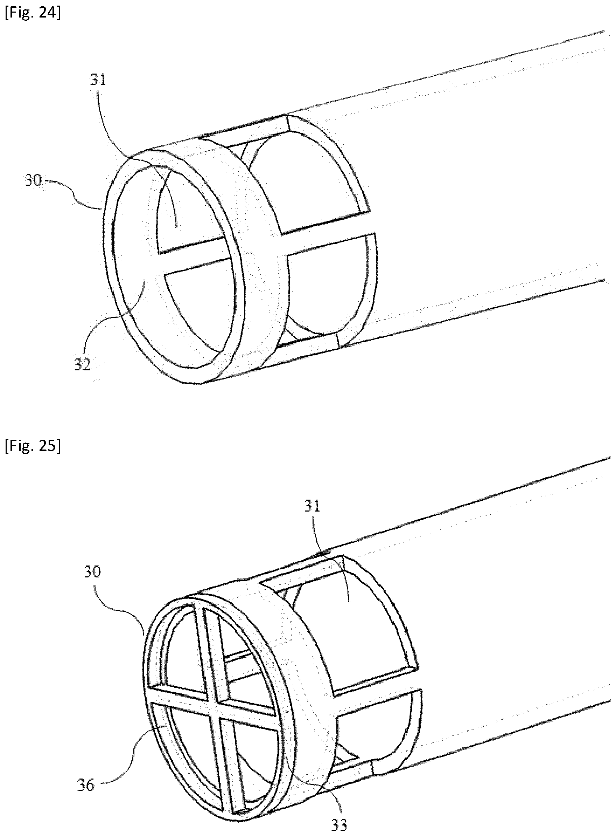

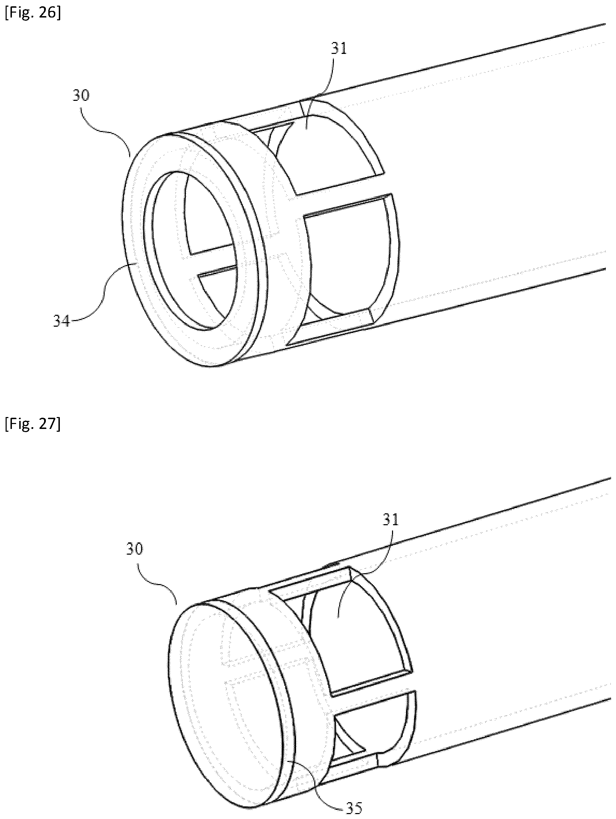

The structure of the distal end of the micro catheter device 2 is shown in Figs. 24 to 27. The distal end head 30 of the micro catheter device 2 can optionally contain a non occluded head 32, or a semi occluded limit structure 33, 34, or a fully occluded limit structure 35; wherein the semi occluded limit structure 33 is provided with an opening structure 36.

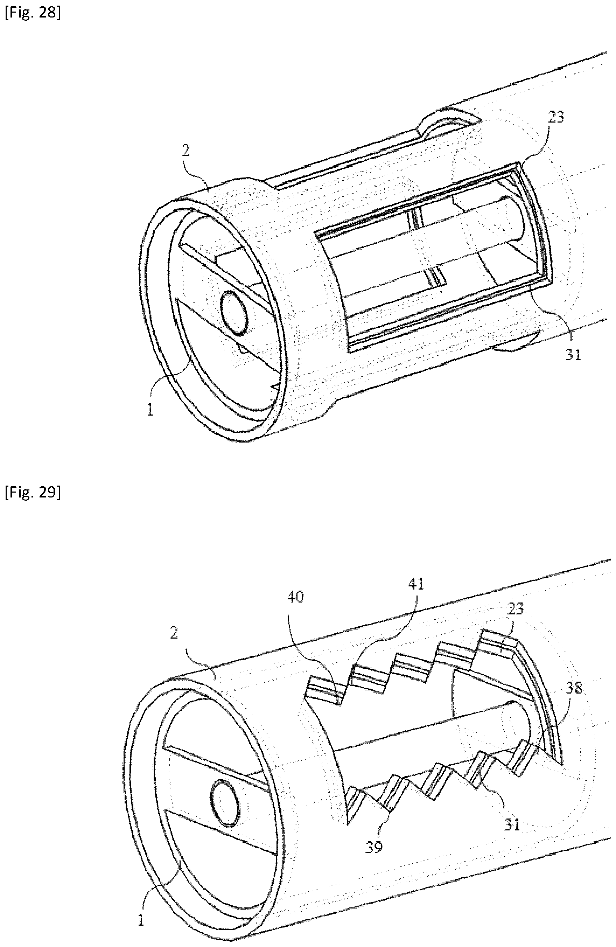

The opening shape of the micro catheter opening 31 of the micro catheter device 2 shown in Figs. 28 to 30 coincides with or corresponds to the opening shape of the rotary grinding head opening 23 of the distal end rotary grinding head device 1, which can be a rectangular or an irregular shape with a serrated edge along the axial direction.

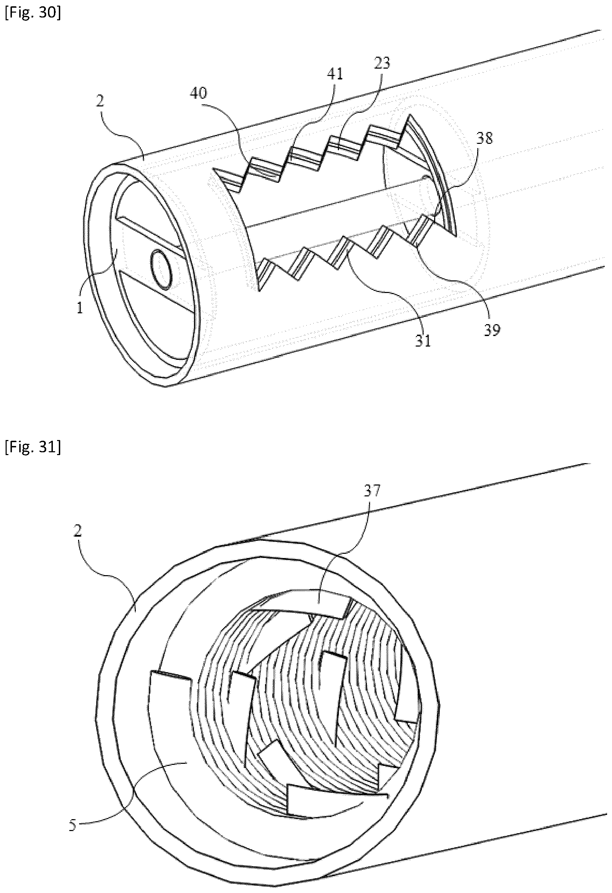

The micro catheter openings 31 of the micro catheter device 2 shown in Figs. 28 to 30 can be rectangular or irregularly shaped with serrated edges along the axial direction. In Fig. 29, during the rotation of the distal end rotary grinding head device, the relative displacement is generated with respect to the micro catheter, and the top point 38 of the serrated edge of the micro catheter opening coincides with the top point 40 of the serrated edge of the rotary grinding head opening of the far-end rotary grinding head device at a certain time, forming a shear force, so as to achieve the effect of thrombus breaking. In Fig. 30, the top point 38 of the serrated edge of the micro catheter opening coincides with the bottom point 41 of the serrated edge of the rotary grinding opening of the distal end rotary grinding device at a certain time to form a shear force.

Fig. 31 shows the structure of the distal end of the micro catheter device 2 according to an embodiment of the present invention, wherein the connection mandrel 5 is provided with a rotary cutting head 37 at the inner circumferentially range, which can be blade shaped or triangular tapered; the connection mandrel 5 and the rotary cutting head 37 are fixed together by laser welding or adhesive.

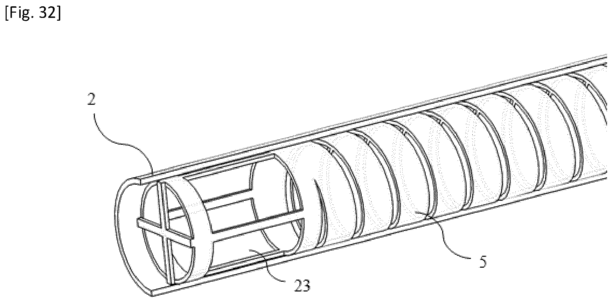

Fig. 32 shows the structure of the distal end of the micro catheter device 2 according to an embodiment of the present invention, wherein the connection mandrel 5 is formed with a single helix notch by laser cutting a metal tube , which makes the connection mandrel 5 have good flexibility; at the diatal end of the connection mandrel 5, the laser cutting method is used to cut the rotary grinding head opening 23, such that the connection mandrel 5 has the function of rotary cutting.

In actual use of the present apparatus, the operator chooses the vascular access to the affected area and sends a guide wire along the vascular access to the affected area; the micro catheter passes through the guide wire, and under the guidance of the guide wire, the distal end of the micro catheter reaches the position of the vascular obstruction, and then withdraw the guide wire. Put the distal end rotary grinding head device integrated in the handle into the micro catheter, grip the connection mandrel and push it to the distal end of the vascular access until the micro catheter and the handle form a whole through quick connection of interfaces. The operator can adjust the position of the distal end rotary grinding head device, such that the distal end rotary grinding head device cooperates with the micro catheter opening of the micro catheter device, so as to achieve better rotary cutting effect. At this time, the operator can operate the handle for aspiration, at the same time, the motor drives the head of the distal end rotary grinding head device to rotate rapidly via the connection mandrel, at the same time, it can continue to adjust the position of the distal end rotary grinding head device, which can exceed the length range of the micro catheter device to directly rotary grinding the thrombus. The thrombus in the blood vessels is broken and aspirated inside and collected outside the human body. This equipment is suitable for rapid removal of embolization in intracranial, coronary and peripheral vessels.

The foregoing description of the specific exemplary embodiments of the invention is for the purpose of explanation and description. The foregoing description does not want to be exhaustive, nor to limit the invention to the precise form disclosed. Obviously, many changes and variations are possible according to the above teaching. The purpose of selecting and describing exemplary embodiments is to explain the specific principles and practical applications of the invention, so that other skilled persons in the art can realize and utilize various exemplary embodiments of the invention and various selection forms and modification forms. The scope of the invention is intended to be defined by the appended claims.

## Claims

1. An apparatus for breaking thrombus and aspirating the thrombus, the apparatus comprising:
an aspirating rotary grinding hand-held system (6) which integrates a rotary grinding head driving device (4), an aspirating device (3), a power supply device (8) and a control device (9);
a micro catheter device (2) comprising a micro catheter, which is communicated with the aspirating device (3) and the rotary grinding head driving device (4), respectively; a waste liquid collecting device (7) which is connected to the aspirating device (3), wherein the aspirating device (3) and the waste liquid collecting device (7) form an airtight passage; and
a distal end rotary grinding head device (1) which is connected to the rotary grinding head driving device (4) through a connection mandrel (5), wherein the power supply device (8) provides power for the rotary grinding head driving device (4);
wherein the distal end rotary grinding head device (1) and the connection mandrel (5) are accommodated in an inner cavity of the micro catheter device (2); and
wherein the control device (9) controls the aspirating device (3) and the rotary grinding head driving device (4), such that the aspirating device (3) and the rotary grinding head driving device (4) run simultaneously,
the apparatus being **characterized in that** the distal end rotary grinding head device (1) is cylindrical-shaped or shuttle-shaped and has a length comprised between 0,5 millimeters and 15 millimeters, **in that** the distal end rotary grinding head device (1) comprises a side surface provided with rotary grinding head openings (23), **in that** the connection mandrel (5) is composed of a core wire (46) having a periphery, a protective sheath tube (45) being sheathed on said periphery, and **in that** the protective sheath tube (45) comprises a surface provided with a spiral cutting pattern (47) .

2. The apparatus for breaking thrombus and aspirating the thrombus according to claim 1, wherein the rotary grinding head driving device (4) and the aspirating device (3) are mechanically or electrically connected to realize simultaneous operation and can stop operation at the same time.

3. The apparatus for breaking thrombus and aspirating the thrombus according to claim 1, wherein the distal end rotary grinding head device (1) is made of polymer material or metal material with good biocompatibility.

4. The apparatus for breaking thrombus and aspirating the thrombus according to claim 1, wherein the distal end rotary grinding head device (1) can slide finitely within the micro catheter (2), and the sliding distance of the distal end rotary grinding head device (1) is between 1 mm and 50 mm.

5. The apparatus for breaking thrombus and aspirating the thrombus according to claim 1, wherein the distal end rotary grinding head device (1) can exceed the length range of the micro catheter device (2), and the length beyond the micro catheter device (2) is 0.5 mm to 5 mm.

6. The apparatus for breaking thrombus and aspirating the thrombus according to claim 1, wherein the distal end rotary grinding head device (1) is integrally formed or formed by connecting a plurality of components.

7. The apparatus for breaking thrombus and aspirating the thrombus according to claim 1, wherein the number of the rotary grinding head openings (23) of the distal end rotary grinding head device (1) is 1 to 15.

8. The apparatus for breaking thrombus and aspirating the thrombus according to claim 1, wherein the rotary grinding head opening (23) of the distal end rotary grinding head device (1) is a rectangular hole, or a hole having a serrated edge, or an elliptical hole.

9. The apparatus for breaking thrombus and aspirating the thrombus according to claim 1, wherein the micro catheter device (2) is made of a polymer material having good biocompatibility, or a composite material of a polymer material and a metal material, or a polymer pipe sleeved with a metal material.

10. The apparatus for breaking thrombus and aspirating the thrombus according to claim 1, wherein the metal material included in the micro catheter device (2) is hard alloy material and/or super elastic alloy material.

11. The apparatus for breaking thrombus and aspirating the thrombus according to claim 1, wherein the distal end position (30) of the micro catheter device (2) entering the human blood vessel is non occluded (32), or is provided with a semi occluded limit structure (33, 34), or is provided with a fully occluded limit structure (35).

12. The apparatus for breaking thrombus and aspirating the thrombus according to claim 11, wherein the semi occluded limit structure (33, 34) and the fully occluded limit structure (35) are polymer materials, or hard alloy materials, or composite materials composed of polymer materials and hard alloy materials.

13. The apparatus for breaking thrombus and aspirating the thrombus according to claim 11, wherein the number of openings (36) of the semi occluded limit structure (33, 34) is 1 to 15.

14. The apparatus for breaking thrombus and aspirating the thrombus according to claim 1, wherein the side surface of the micro catheter device is provided with micro catheter openings (31) close to the distal end position.

15. The apparatus for breaking thrombus and aspirating the thrombus according to claim 14, wherein the number of the micro catheter openings (31) of the micro catheter device (2) is 1 to 15.

16. The apparatus for breaking thrombus and aspirating the thrombus according to claim 14, wherein the distance from the distal end of the micro catheter openings (31) of the micro catheter device (2) to the distal end of the micro catheter device (2) is 0.1 mm to 15 mm.

17. The apparatus for breaking thrombus and aspirating the thrombus according to claim 14, wherein the micro catheter opening (31) of the micro catheter device (2) is a rectangular hole, or a hole having a serrated edge, or an elliptical hole.

18. The apparatus for breaking thrombus and aspirating the thrombus according to claim 1, wherein the periphery of the connection mandrel (5) is provided with 2 to 15 stabilizers (44).

19. The apparatus for breaking thrombus and aspirating the thrombus according to claim 18, wherein the width of the stabilizer (44) is from 1 mm to 3 mm.

20. The apparatus for breaking thrombus and aspirating the thrombus according to claim 1, wherein the materials of the core wire (46) and the protective sheath tube (45) are hard metal alloy material, super elastic alloy material or polymer material.

21. The apparatus for breaking thrombus and aspirating the thrombus according to claim 1, wherein the core wire (46) rotates at the same speed as the protective sheath tube (45); or, the core wire (46) rotates while the protective sheath tube (45) is in stationary.

22. The apparatus for breaking thrombus and aspirating the thrombus according to claim 1, wherein the core wire (46) is longer than the protective sheath tube (45), and the length difference between the two is 2 mm to 150 mm.

23. The apparatus for breaking thrombus and aspirating the thrombus according to claim 1, wherein the core wire (46) is composed of a single thread or a plurality of threads, and the core wire (46) composed of a plurality of threads is formed by spiraling a plurality of threads or by spiraling a plurality of threads around a thread.

24. The apparatus for breaking thrombus and aspirating the thrombus according to claim 23, wherein the core wire (46) is composed of 2 to 12 threads.

25. The apparatus for breaking thrombus and aspirating the thrombus according to claim 1, wherein when the rotary grinding head opening (21) of the distal end rotary grinding head device (1) is a hole having a serrated edge and the micro catheter opening (31) of the micro catheter device (2) is also a hole having a serrated edge, during the rotation process, the top point (38) of the serrated edge of the micro catheter opening coincides with the bottom point (41) of the serrated edge of the rotary grinding head opening at a certain position, or the top point (38) of the serrated edge of the micro catheter opening coincides with the top point (40) of the serrated edge of the rotary grinding head opening at a certain position.

## Patentansprüche

1. Einrichtung zum Öffnen eines Thrombus und Absaugen des Thrombus, wobei die Einrichtung Folgendes umfasst:
ein tragbares Drehschleifabsaugsystem (6), in das eine Drehschleifkopfantriebsvorrichtung (4), eine Absaugvorrichtung (3), eine Stromversorgungsvorrichtung (8) und eine Steuervorrichtung (9) integriert sind;
eine Mikrokathetervorrichtung (2), die einen Mikrokatheter umfasst, der mit der Absaugvorrichtung (3) bzw. der Drehschleifkopfantriebsvorrichtung (4) kommuniziert;
eine Abfallflüssigkeitssammelvorrichtung (7), die mit der Absaugvorrichtung (3) verbunden ist, wobei die Absaugvorrichtung (3) und die Abfallflüssigkeitssammelvorrichtung (7) eine luftdichte Durchleitung bilden; und
eine Distalendedrehschleifkopfvorrichtung (1), die über einen Verbindungsdorn (5) mit der Drehschleifkopfantriebsvorrichtung (4) verbunden ist, wobei die Stromversorgungsvorrichtung (8) Strom für die Drehschleifkopfantriebsvorrichtung (4) bereitstellt;
wobei die Distalendedrehschleifkopfvorrichtung (1) und der Verbindungsdorn (5) in einem inneren Hohlraum der Mikrokathetervorrichtung (2) aufgenommen sind; und
wobei die Steuervorrichtung (9) die Absaugvorrichtung (3) und die Drehschleifkopfantriebsvorrichtung (4) derart steuert, dass die Absaugvorrichtung (3) und die Drehschleifkopfantriebsvorrichtung (4) gleichzeitig laufen,
wobei die Einrichtung **dadurch gekennzeichnet ist, dass** die Distalendedrehschleifkopfvorrichtung (1) zylinderförmig oder schiffchenförmig ist und eine Länge aufweist, die zwischen 0,5 Millimeter und 15 Millimeter umfasst ist, dadurch, dass die Distalendedrehschleifkopfvorrichtung (1) eine Seitenfläche umfasst, die mit Drehschleifkopföffnungen (23) versehen ist, dadurch, dass der Verbindungsdorn (5) aus einem Kerndraht (46) mit einer Peripherie besteht, wobei die Peripherie von einem Schutzmantelrohr (45) umhüllt ist, und dadurch, dass das Schutzmantelrohr (45) eine Fläche aufweist, die mit einem Spiralschneidemuster (47) versehen ist.

2. Einrichtung zum Öffnen eines Thrombus und Absaugen des Thrombus nach Anspruch 1, wobei die Drehschleifkopfantriebsvorrichtung (4) und die Absaugvorrichtung (3) mechanisch oder elektrisch verbunden sind, um einen gleichzeitigen Betrieb zu realisieren, und den Betrieb zur selben Zeit beenden können.

3. Einrichtung zum Öffnen eines Thrombus und Absaugen des Thrombus nach Anspruch 1, wobei die Distalendedrehschleifkopfvorrichtung (1) aus einem Polymermaterial oder einem Metallmaterial mit guter Biokompatibilität besteht.

4. Einrichtung zum Öffnen eines Thrombus und Absaugen des Thrombus nach Anspruch 1, wobei die Distalendedrehschleifkopfvorrichtung (1) endlich im Mikrokatheter (2) gleiten kann und der Gleitabstand der Distalendedrehschleifkopfvorrichtung (1) zwischen 1 mm und 50 mm beträgt.

5. Einrichtung zum Öffnen eines Thrombus und Absaugen des Thrombus nach Anspruch 1, wobei die Distalendedrehschleifkopfvorrichtung (1) den Längenbereich der Mikrokathetervorrichtung (2) überschreiten kann und die Länge hinter der Mikrokathetervorrichtung (2) 0,5 mm bis 5 mm beträgt.

6. Einrichtung zum Öffnen eines Thrombus und Absaugen des Thrombus nach Anspruch 1, wobei die Distalendedrehschleifkopfvorrichtung (1) integral gebildet oder durch Verbinden einer Vielzahl von Komponenten gebildet ist.

7. Einrichtung zum Öffnen eines Thrombus und Absaugen des Thrombus nach Anspruch 1, wobei die Anzahl der Drehschleifkopföffnungen (23) der Distalendedrehschleifkopfvorrichtung (1) 1 bis 15 beträgt.

8. Einrichtung zum Öffnen eines Thrombus und Absaugen des Thrombus nach Anspruch 1, wobei die Drehschleifkopföffnung (23) der Distalendedrehschleifkopfvorrichtung (1) ein rechteckiges Loch oder ein Loch mit einem gezackten Rand oder ein elliptisches Loch ist.

9. Einrichtung zum Öffnen eines Thrombus und Absaugen des Thrombus nach Anspruch 1, wobei die Mikrokathetervorrichtung (2) aus einem Polymermaterial mit guter Biokompatibilität oder einem Verbundmaterial eines Polymermaterials und eines Metallmaterials besteht oder eine Polymerleitung ist, auf die ein Metallmaterial gehülst ist.

10. Einrichtung zum Öffnen eines Thrombus und Absaugen des Thrombus nach Anspruch 1, wobei das Metallmaterial, das in der Mikrokathetervorrichtung (2) beinhaltet ist, ein hartes Legierungsmaterial und/oder ein superelastisches Legierungsmaterial ist.

11. Einrichtung zum Öffnen eines Thrombus und Absaugen des Thrombus nach Anspruch 1, wobei die distale Endposition (30) der Mikrokathetervorrichtung (2), die in das menschliche Blutgefäß eintritt, nicht verschlossen (32) ist oder mit einer halbverschlossenen Begrenzungsstruktur (33, 34) versehen ist oder mit einer ganz verschlossenen Begrenzungsstruktur (35) versehen ist.

12. Einrichtung zum Öffnen eines Thrombus und Absaugen des Thrombus nach Anspruch 11, wobei die halbverschlossene Begrenzungsstruktur (33, 34) und die ganz verschlossene Begrenzungsstruktur (35) Polymermaterialien oder harte Legierungsmaterialien oder Verbundmaterialien, die aus Polymermaterialien und aus harten Legierungsmaterialien bestehen, sind.

13. Einrichtung zum Öffnen eines Thrombus und Absaugen des Thrombus nach Anspruch 11, wobei die Anzahl von Öffnungen (36) der halbverschlossenen Begrenzungsstruktur (33, 34) 1 bis 15 beträgt.

14. Einrichtung zum Öffnen eines Thrombus und Absaugen des Thrombus nach Anspruch 1, wobei die Seitenfläche der Mikrokathetervorrichtung nahe der distalen Endposition mit Mikrokatheteröffnungen (31) versehen ist.

15. Einrichtung zum Öffnen eines Thrombus und Absaugen des Thrombus nach Anspruch 14, wobei die Anzahl von Mikrokatheteröffnungen (31) der Mikrokathetervorrichtung (2) 1 bis 15 beträgt.

16. Einrichtung zum Öffnen eines Thrombus und Absaugen des Thrombus nach Anspruch 14, wobei der Abstand vom distalen Ende der Mikrokatheteröffnungen (31) der Mikrokathetervorrichtung (2) zum distalen Ende der Mikrokathetervorrichtung (2) 0,1 mm bis 15 mm beträgt.

17. Einrichtung zum Öffnen eines Thrombus und Absaugen des Thrombus nach Anspruch 14, wobei die Mikrokatheteröffnung (31) der Mikrokathetervorrichtung (2) ein rechteckiges Loch oder ein Loch mit einem gezackten Rand oder ein elliptisches Loch ist.

18. Einrichtung zum Öffnen eines Thrombus und Absaugen des Thrombus nach Anspruch 1, wobei die Peripherie des Verbindungsdorns (5) mit 2 bis 15 Stabilisatoren (44) versehen ist.

19. Einrichtung zum Öffnen eines Thrombus und Absaugen des Thrombus nach Anspruch 18, wobei die Breite des Stabilisators (44) von 1 mm bis 3 mm beträgt.

20. Einrichtung zum Öffnen eines Thrombus und Absaugen des Thrombus nach Anspruch 1, wobei die Materialien des Kerndrahts (46) und des Schutzmantelrohrs (45) ein hartes Metalllegierungsmaterial, ein superelastisches Legierungsmaterial oder ein Polymermaterial sind.

21. Einrichtung zum Öffnen eines Thrombus und Absaugen des Thrombus nach Anspruch 1, wobei sich der Kerndraht (46) mit derselben Geschwindigkeit wie das Schutzmantelrohr (45) dreht oder sich der Kerndraht (46) dreht, während das Schutzmantelrohr (45) stationär ist.

22. Einrichtung zum Öffnen eines Thrombus und Absaugen des Thrombus nach Anspruch 1, wobei der Kerndraht (46) länger als das Schutzmantelrohr (45) ist und die Längendifferenz zwischen den beiden 2 mm bis 150 mm beträgt.

23. Einrichtung zum Öffnen eines Thrombus und Absaugen des Thrombus nach Anspruch 1, wobei der Kerndraht (46) aus einem einzelnen Strang oder einer Vielzahl von Strängen besteht und der Kerndraht (46), der aus einer Vielzahl von Strängen besteht, durch spiralförmiges Verdrehen einer Vielzahl von Strängen oder durch spiralförmiges Verdrehen einer Vielzahl von Strängen um einen Strang gebildet ist.

24. Einrichtung zum Öffnen eines Thrombus und Absaugen des Thrombus nach Anspruch 23, wobei der Kerndraht (46) aus 2 bis 12 Strängen besteht.

25. Einrichtung zum Öffnen eines Thrombus und Absaugen des Thrombus nach Anspruch 1, wobei, wenn die Drehschleifkopföffnung (21) der Distalendedrehschleifkopfvorrichtung (1) ein Loch mit einem gezackten Rand ist und die Mikrokatheteröffnung (31) der Mikrokathetervorrichtung (2) ebenfalls ein Loch mit einem gezackten Rand ist, die obere Stelle (38) des gezackten Randes der Mikrokatheteröffnung während des Drehprozesses sich in einer bestimmten Position mit der unteren Stelle (41) des gezackten Randes der Drehschleifkopföffnung deckt oder sich die obere Stelle (38) des gezackten Randes der Mikrokatheteröffnung in einer bestimmten Position mit der oberen Stelle (40) des gezackten Randes der Drehschleifkopföffnung deckt.

## Revendications

1. Un appareil pour briser un thrombus et aspirer le thrombus, l'appareil comprenant :
un système manuel de meulage rotatif par aspiration (6) qui intègre un dispositif d'entraînement de tête de meulage rotatif (4), un dispositif d'aspiration (3), un dispositif d'alimentation en énergie (8) et un dispositif de commande (9) ;
un dispositif de micro-cathéter (2) comprenant un micro-cathéter, qui est en communication avec le dispositif d'aspiration (3) et le dispositif d'entrainement de la tête de meulage rotative (4), respectivement ; un dispositif de collecte de liquide usé (7) qui est relié au dispositif d'aspiration (3), dans lequel le dispositif d'aspiration (3) et le dispositif de collecte de liquide usé (7) forment un passage étanche à l'air ; et
un dispositif de tête de meulage rotatif à extrémité distale (1) qui est connecté au dispositif d'entraînement de tête de meulage rotatif (4) par l'intermédiaire d'un mandrin de connexion (5), dans lequel le dispositif d'alimentation en énergie (8) fournit de l'énergie au dispositif d'entraînement de tête de meulage rotatif (4) ;
dans lequel le dispositif de tête de meulage rotatif à extrémité distale (1) et le mandrin de connexion (5) sont logés dans une cavité interne du dispositif de micro-cathéter (2) ; et
dans lequel le dispositif de commande (9) commande le dispositif d'aspiration (3) et le dispositif d'entraînement de la tête de meulage rotative (4), de sorte que le dispositif d'aspiration (3) et le dispositif d'entraînement de la tête de meulage rotative (4) fonctionnent simultanément,
l'appareil étant **caractérisé en ce que** le dispositif de tête de broyage rotatif à extrémité distale (1) est en forme de cylindre ou de navette et a une longueur comprise entre 0,5 millimètre et 15 millimètres, **en ce que** le dispositif de tête de broyage rotatif à extrémité distale (1) comprend une surface latérale munie d'ouvertures de tête de broyage rotatif (23), **en ce que** le mandrin de connexion (5) est composé d'un fil central (46) ayant une périphérie, un tube de gaine protectrice (45) étant gainé sur ladite périphérie, et **en ce que** le tube de gaine protectrice (45) comprend une surface munie d'un motif de coupe en spirale (47).

2. Appareil pour rompre un thrombus et aspirer le thrombus selon la revendication 1, dans lequel le dispositif d'entra nement de la tête de meulage rotative (4) et le dispositif d'aspiration (3) sont reliés mécaniquement ou électriquement pour réaliser un fonctionnement simultané et peuvent arrêter le fonctionnement en même temps.

3. Appareil pour briser un thrombus et aspirer le thrombus selon la revendication 1, dans lequel le dispositif de tête de broyage rotatif à extrémité distale (1) est fait d'un matériau polymère ou d'un matériau métallique avec une bonne biocompatibilité.

4. Appareil pour rompre un thrombus et aspirer le thrombus selon la revendication 1, dans lequel le dispositif de tête de broyage rotatif à extrémité distale (1) peut coulisser finement à l'intérieur du micro cathéter (2), et la distance de coulissement du dispositif de tête de broyage rotatif à extrémité distale (1) est comprise entre 1 mm et 50 mm.

5. Appareil pour briser un thrombus et aspirer le thrombus selon la revendication 1, dans lequel le dispositif de tête de meulage rotatif à extrémité distale (1) peut dépasser la plage de longueur du dispositif de microcathéter (2), et la longueur au-delà du dispositif de microcathéter (2) est de 0,5 mm à 5 mm.

6. Appareil pour briser un thrombus et aspirer le thrombus selon la revendication 1, dans lequel le dispositif de tête de broyage rotatif à extrémité distale (1) est formé d'un seul tenant ou formé en reliant une pluralité de composants.

7. Appareil pour rompre un thrombus et aspirer le thrombus selon la revendication 1, dans lequel le nombre d'ouvertures de la tête de broyage rotative (23) du dispositif de tête de broyage rotative d'extrémité distale (1) est de 1 à 15.

8. Appareil pour briser un thrombus et aspirer le thrombus selon la revendication 1, dans lequel l'ouverture de la tête de broyage rotative (23) du dispositif de tête de broyage rotative d'extrémité distale (1) est un trou rectangulaire, ou un trou ayant un bord dentelé, ou un trou elliptique.

9. Appareil pour rompre un thrombus et aspirer le thrombus selon la revendication 1, dans lequel le dispositif de micro-cathéter (2) est fait d'un matériau polymère ayant une bonne biocompatibilité, ou d'un matériau composite d'un matériau polymère et d'un matériau métallique, ou d'un tube polymère gainé d'un matériau métallique.

10. Appareil pour briser un thrombus et aspirer le thrombus selon la revendication 1, dans lequel le matériau métallique inclus dans le dispositif de micro cathéter (2) est un matériau d'alliage dur et/ou un matériau d'alliage super élastique.

11. Appareil pour rompre un thrombus et aspirer le thrombus selon la revendication 1, dans lequel la position d'extrémité distale (30) du dispositif de microcathéter (2) pénétrant dans le vaisseau sanguin humain est non occluse (32), ou est pourvue d'une structure limite semi-occluse (33, 34), ou est pourvue d'une structure limite totalement occluse (35).

12. Appareil pour rompre un thrombus et aspirer le thrombus selon la revendication 11, dans lequel la structure limite semi-occluse (33, 34) et la structure limite totalement occluse (35) sont des matériaux polymères, ou des matériaux en alliage dur, ou des matériaux composites composés de matériaux polymères et de matériaux en alliage dur.

13. Appareil pour rompre un thrombus et aspirer le thrombus selon la revendication 11, dans lequel le nombre d'ouvertures (36) de la structure limite semi-occluse (33, 34) est de 1 à 15.

14. Appareil pour rompre un thrombus et aspirer le thrombus selon la revendication 1, dans lequel la surface latérale du dispositif de micro-cathéter est pourvue d'ouvertures de microcathéter (31) à proximité de la position d'extrémité distale.

15. Appareil pour rompre un thrombus et aspirer le thrombus selon la revendication 14, dans lequel le nombre d'ouvertures de micro-cathéter (31) du dispositif de micro-cathéter (2) est de 1 à 15.

16. Appareil pour rompre un thrombus et aspirer le thrombus selon la revendication 14, dans lequel la distance entre l'extrémité distale des ouvertures de micro-cathéter (31) du dispositif de microcathéter (2) et l'extrémité distale du dispositif de micro-cathéter (2) est de 0,1 mm à 15 mm.

17. Appareil pour rompre un thrombus et aspirer le thrombus selon la revendication 14, dans lequel l'ouverture de micro-cathéter (31) du dispositif de micro-cathéter (2) est un trou rectangulaire, ou un trou ayant un bord dentelé, ou un trou elliptique.

18. Appareil de rupture de thrombus et d'aspiration du thrombus selon la revendication 1, dans lequel la périphérie du mandrin de connexion (5) est pourvue de 2 à 15 stabilisateurs (44).

19. Appareil pour briser un thrombus et aspirer le thrombus selon la revendication 18, dans lequel la largeur du stabilisateur (44) est de 1 mm à 3 mm.

20. Appareil pour rompre un thrombus et aspirer le thrombus selon la revendication 1, dans lequel les matériaux du fil central (46) et du tube de gaine protectrice (45) sont un matériau d'alliage de métal dur, un matériau d'alliage super élastique ou un matériau polymère.

21. Appareil pour rompre un thrombus et aspirer le thrombus selon la revendication 1, dans lequel le fil central (46) tourne à la même vitesse que le tube de gaine protectrice (45) ; ou, le fil central (46) tourne alors que le tube de gaine protectrice (45) est immobile.

22. Appareil pour rompre un thrombus et aspirer le thrombus selon la revendication 1, dans lequel le fil central (46) est plus long que le tube de gaine protectrice (45), et la différence de longueur entre les deux est de 2 mm à 150 mm.

23. Appareil pour rompre un thrombus et aspirer le thrombus selon la revendication 1, dans lequel le fil central (46) est composé d'un seul fil ou d'une pluralité de fils, et le fil central (46) composé d'une pluralité de fils est formé en spiralant une pluralité de fils ou en spiralant une pluralité de fils autour d'un fil.

24. Appareil pour briser un thrombus et aspirer le thrombus selon la revendication 23, dans lequel le fil central (46) est composé de 2 à 12 fils.

25. Appareil pour briser un thrombus et aspirer le thrombus selon la revendication 1, dans lequel, lorsque l'ouverture de la tête de meulage rotative (21) du dispositif de tête de meulage rotative d'extrémité distale (1) est un trou ayant un bord dentelé et l'ouverture du micro-cathéter (31) du dispositif de micro-cathéter (2) est également un trou ayant un bord dentelé, pendant le processus de rotation, le point supérieur (38) du bord dentelé de l'ouverture du micro cathéter coïncide avec le point inférieur (41) du bord dentelé de l'ouverture de la tête de meulage rotative à une certaine position, ou le point supérieur (38) du bord dentelé de l'ouverture du micro cathéter coïncide avec le point supérieur (40) du bord dentelé de l'ouverture de la tête de meulage rotative à une certaine position.
